# EUROPEAN PATENT SPECIFICATION

(11) **EP 0 438 566 B1**
(45) Date of publication and mention of the grant of the patent: **25.09.1996**
(21) Application number: 90912128.7
(22) Date of filing: 16.08.1990
(51) Int. Cl.: C07K 7/02, C07K 7/22, A61K 38/08

(54) **Substance P antagonists**
Substance P Antagoniste
Antagonistes de substance P

(30) Priority: 16.08.1989 US 394727
(43) Date of publication of application: 31.07.1991
(73) Proprietor: The Administrators of The Tulane Educational Fund, New Orleans Louisiana 70112 (US)
(72) Inventor: COY, David, H., New Orleans, LA 70115 (US); MOREAU, Jacques-Pierre, Upton, MA 01568 (US)
(74) Representative: Deans, Michael John Percy
(86) International application number: US9004633
(87) International publication number: WO9102745

(56) References cited:
- EP-A- 0 347 802
- US-A- 3 862 114
- US-A- 4 439 360
- US-A- 4 481 139
- US-A- 4 803 261
- J.E.RIVIER ET AL 'PEPTIDES, Chemistry, Structure and Biology, Proceedings of the Eleventh American Peptide Symposium, July 9-14, 1989, La Jolla, California, U.S.A.' 1990 , ESCOM , LEIDEN
- S.L.Harbeson et al, Synthesis and biological activity of (CH2NH) analogs of neurokinin A(4-10)
- TETRAHEDRON, (INCL. TETRAHEDRON REPORTS) vol. 44, no. 3, 1988, OXFORD GB pages 835 - 841 D.H.COY ET AL 'SOLID PHASE REDUCTIVE ALKYLATION TECHNIQUES IN ANALOGUE PEPTIDE BOND AND SIDECHAIN MODIFICATION'
- AMERICAN JOURNAL OF PHYSIOLOGY: GASTROINTESTINAL AND LIVER PHYSIOLOGY vol. 17, no. 6, June 1988, pages G883 - G890 R.T.JENSEN ET AL 'Characterization of ability of various substance P antagonists to inhibit action of bombesin.'
- JOURNAL OF BIOLOGICAL CHEMISTRY. vol. 264, no. 28, 5 October 1989, BALTIMORE US pages 16667 - 16671 J-M QIAN ET AL 'Reduced Peptide Bond pseudopeptide Analogues of Substance P'

## Description

This invention relates to therapeutic peptides, in particular analogs of the naturally occurring peptide substance P.

Substance P (SP) has numerous pharmacologic effects including vasodilation and hypotension, contraction of non-vascular smooth muscle, stimulation of salivary and pancreatic secretion, depolarization of various neurons and histamine release from mast cells. SP is thought to play a variety of physiological roles (many of which are associated with the induction of pain). These include regulation of peristalsis and smooth muscle activity in the gastrointestinal tract, regulation of salivary and pancreatic secretion, regulation of the inflammatory response to peripheral tissue injury, neurotransmission, and regulation of neuroimmunomodulation. Mantyh et al. (1989) Proc. Natl. Acad. Sci. USA 86:5193 reports the presence of substance P receptors at wound sites in the central nervous system and suggests that SP may be involved in regulating the response to injury in the central nervous system as well as in peripheral tissues. Substance P is also a proliferative agent, stimulating the proliferation of fibroblasts, T-lymphocytes, endothelial cells, smooth muscle cells and astrocytes.

SP belongs to a family of bioactive peptides known as the tachykinins. The structure, activity, and function of SP and other tachykinins are discussed in Payan (1989) Ann. Rev. Med. 40:341. As discussed in Payan, SP shares common pharmacological properties and a conserved carboxyl terminal sequence (Phe-X-Gly-Leu-Met-NH₂, where X is a branched aliphatic or aromatic amino acid residue) with the other tachykinins. The principal biological activities, and the ability to bind to a receptor, reside in the carboxyl terminal sequence of these peptides. Selectivity toward a specific tachykinin receptor is determined by the amino-terminal sequence of the peptides Iverson et al., (1989) The Tachykinin System, Abstract presented at the 11th American Peptide Symposium. The conservation of carboxyl terminal sequence extends beyond SP and other mammalian tachykinins to other bioactive peptides, as shown in Table 1.

Numerous derivatives of SP, made by side chain modification and/or D-amino acid substitution, have been shown to act as SP receptor antagonists. Folkers, U.S. Patent No. 4,481,139, describes substance P antagonists made by D or L amino acid substitution. These antagonists include the undecapeptide analog spantide (D-Arg-Pro-Lys-Pro-Gln-Gln-D-Trp- Phe-D-Trp-Leu-Leu-NH₂) as well as truncated analogs of substance P. Jensen et al. (1988) Am. J. of Physiol. 254: G883 characterized the ability of various SP antagonists to inhibit the action of bombesin. Jensen et al. studied four SP analogues: Arg-D-Pro-Lys-Pro-Gln-Gln-D-Phe-Phe-D-Trp-Leu-Met-NH₂; Arg-D-Pro-Lys-Pro-Gln-Gln-D-Trp-Phe-D-Trp-Leu-Met-NH₂; D-Arg-D-Pro-Lys-Pro-Gln-Gln-D-Trp-Phe-D-Trp-Leu-Leu-NH₂; and D-Arg-Pro-Lys-Pro-Gln-Gln-D-Trp-Phe-D-Trp-Leu-Leu-NH₂. Jensen et al. also studied two SP analogues with the first 3 amino acid residues deleted, D-Pro-Gln-Gln-D-Trp-Phe-D-Trp-Leu-Met-NH₂ and D-Pro-Gln-Gln-D-Trp-Phe-D-Trp-D-Trp-Met-NH₂. None of these receptor binding peptides are, however, specific to the SP receptor. All were found to inhibit bombesin-stimulated amylase release. Jensen et al. concluded that "the ability to inhibit the action of bombesin is a general property of SP analogues that also function as SP receptor antagonists and that the SP receptor antagonists are each inhibiting the action of bombesin by functioning as bombesin receptor antagonists."

Woll et al. (1988) Proc. Nat. Acad. Sci. USA 85: 1857 found the substance P antagonist D-Arg-Pro-Lys-Pro-D-Phe-Gln- D-Trp-Phe-D-Trp-Leu-Leu-NH₂ a potent bombesin antagonist in murine Swiss 3T3 cells.

Agonists and antagonists of a wide spectrum of biologically active peptide hormones, including substance P, have been synthesized by the introduction of modification of the peptide bonds of the peptide hormone, see Spatola (1983) in Chemistry and Biochemistry of Amino Acids, Peptides, and Proteins (B. Weinstein, ed.) M. Dekker, New York and Basel, pp. 267-357, for a recent review of the field.

We employ the following uncommon abbreviations herein:- 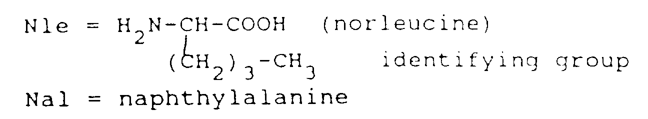 SP=Arg-Pro-Lys-Pro-Gln-Gln-Phe-Phe-Gly-Leu-Met-NH₂ (substance P).

According to a first aspect of this invention, we provide a substance P analog of the formula: 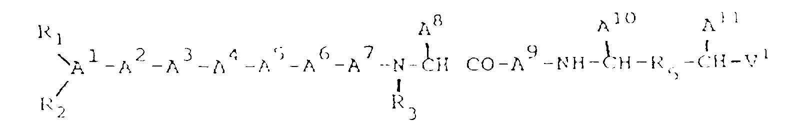 wherein
- A¹ =: the D or L isomer of Arg or Lys;
- A² =: the D or L isomer of Pro;
- A³ =: the D or L isomer of Lys;
- A⁴ =: the D or L isomer of Pro;
- A⁵ =: the D or L isomer of Gln;
- A⁶ =: the D or L isomer of Gln;
- A⁷ =: the D or L isomer of Phe, Trp, β-Nal, o-X-Phe in which X is F, Cl, Br, NO₂, OH, or CH₃, or of p-X-Phe in which X is F, Cl, Br, NO₂, OH, or CH₃;
- A⁸ =: the identifying group of the D or L isomer of Trp_{,} β-Nal, Phe, o-X-Phe in which X is F, Cl, Br, NO₂, OH, or CH₃, or of p-X-Phe in which X is F, Cl, Br, NO₂, OH, or CH₃;
- A⁹ =: Gly or the D or L isomer of Trp, β-Nal, Phe, o-X-Phe in which X is F, Cl, Br, NO₂, OH, or CH₃, or of p-X-Phe in which X is F, Cl, Br, NO₂, OH, or CH₃;
- A¹⁰ =: the identifying group of the D or L isomer of Leu, Nle, Ala, Val or of Ile;
- A¹¹ =: the identifying group of the D or L isomer of Leu, Nle, Ala, Val or of Ile;
- R₃: is H or C₁₋₁₂ alkyl; R₅ is CH₂-NH; and V¹ is 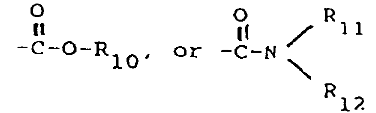 in which each R₁₀, R₁₁, and R₁₂ independently, is H, C₁₋₁₂ alkyl, C₇₋₁₀ phenylalkyl, or C₁₂₋₂₀ naphthylalkyl; and R₁ and R₂, which are bonded to the nitrogen of the α-amino group at the N-terminal amino acid are, independently, H, C₁₋₁₂ alkyl, C₇₋₁₀ phenylalkyl, or COE₁₄ with E₁₄ being C₁₋₂₀ alkyl, C₃₋₂₀ alkenyl, C₃₋₂₀ alkynyl, phenyl, naphthyl, or C₇₋₁₀ phenylalkyl, provided that when one of R₁ or R₂ is COE₁₄, the other must be a hydrogen;
or a pharmaceutically acceptable salt thereof.

In a second and alternative aspect of this invention, there is provided a substance P analog of the formula: 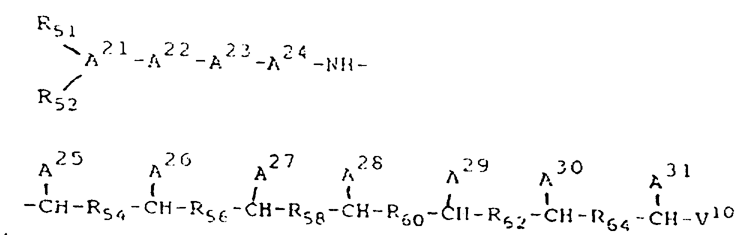 wherein
- A²¹ =: the D or L isomer of Arg, or Lys;
- A²² =: the D or L isomer of Pro;
- A²³ =: the D or L isomer of Lys;
- A²⁴ =: the D or L isomer of Pro;
- A²⁵ =: the identifying group of the D or L isomer of Gln;
- A²⁶ =: the identifying group of the D or L isomer of Gln;
- A²⁷ =: the identifying group of D-Trp; or the identifying group of the D or L isomer of Phe;
- A²⁸ =: the identifying group of the D or L isomer of any one of the amino acids β-Nal, Phe, o-X-Phe in which X is F, Cl, Br, NO₂, OH, or CH₃, or p-X-Phe in which X is F, Cl, Br, NO₂, OH, or CH₃;
- A²⁹ =: the identifying group of the amino acid D-trp; or the identifying group of the D or L isomer of Phe;
- A³⁰ =: the identifying group of the D or L isomer of any one of the amino acids Leu, Nle, Ala, Val or Ile;
- A³¹ =: the identifying group of the D or L isomer of any one of the amino acids Leu, Nle, Ala, Val or Ile;
- R₅₄: is CO-NH or CO-NCH₃; each R₅₆, R₆₂, and R₆₄
independently, is any of CO-NH or CH₂-NH; R₅₈ is CO-NR₆₉ in which R₆₉ is H or C₁₋₁₂ alkyl, or CH₂-NH; R₆₀ is CO-NH; and v¹⁰ is 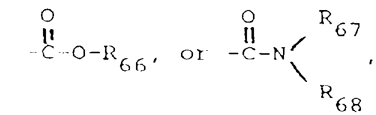 in which each R₆₆, R₆₇, and R₆₈ independently, is H, C₁₋₁₂ alkyl, C₇₋₁₀ phenylalkyl, or C₁₂₋₂₀ naphthylalkyl; and R₅₁ and R₅₂, which are bonded to the nitrogen of the α-omino group at the N-terminal amino acid are, independently, H, C₁₋₁₂ alkyl, C₇₋₁₀ phenylalkyl, or COE₂₄ with E₂₄ being C₁C₂₀ alkyl, C₃₋₂₀ alkenyl, C₃₋₂₀ alkynyl, phenyl, naphthyl, or C₇₋₁₀ phenylalkyl provided that when one of R₅₁ or R₅₂ is COE_{24,} the other is H; with the proviso that at least one of R₅₆, R₅₈, R₆₂, or R₆₄ is CH₂NH; or a pharmaceutically acceptable salt thereof.

We describe below embodiments in which the analog is shown to be capable of acting as a competitive inhibitor of naturally occurring substance P by binding to the receptor and fails to exhibit the in vivo biological activity of the naturally occurring peptide. In preferred embodiments the active site of the linear peptide is in the carboxyl terminal-half of the linear peptide.

By a non-peptide bond is meant that the carbon atom participating in the bond between two residues is reduced from a carbonyl carbon to a methylene carbon, i.e., CH₂-NH; or, less preferably, CH₂-S, CH₂-CH₂, CH₂-CO, or CO-CH₂. (A detailed discussion of the chemistry of non-peptide bonds is given in Coy et al. (1988) Tetrahedron 44,3:835-841, hereby incorporated by reference, Tourwe (1985) Janssen Chim. Acta 3:3-15, 17-18, hereby incorporated by reference, and Spatola (1983) in Chemistry and Biochemistry of Amino Acids, Peptides, and Proteins, (B. Weinstein, ed.) M. Dekker, New York and Basel, pp. 267-357, hereby incorporated by reference.)

The specific SP analogs disclosed below all possess the CH₂-NH pseudopeptide bond.

Preferably, our analogs are 25% homologous, most preferably, 50% homologous, with the naturally occurring peptides.

Examples of preferred substance P analogs are: Arg-Pro-Lys-Pro-Gln-Gln-Phe-Phe-Gly-Leu^{L}[CH₂-NH]Leu-NH₂, D-Arg-Pro-Lys-Pro-Gln-Gln-D-Trp-Phe-D-Trp^{L}[CH₂-NH]Leu-Nle-NH₂, or D-Arg-Pro-Lys-Pro-Gln-Gln-D-Trp-Phe-D-Trp-Leu^{L}[CH₂-NH]Nle-NH₂. (Non-peptide bonds in which the peptide bond is reduced are symbolized herein by "^{L}[CH₂-NH]" or "^{L}"].

Our SP antagonists are useful in the treatment of a patient suffering from diseases involving neurogenic inflamation e.g., rheumatoid arthritis, ulcerative colitis, eczema, and Crohn's disease. Our SP antagonists are useful as antiproliferative agents e.g., in the treatment of small cell lung carcinoma or disorders involving the proliferation of fibroblasts. The antiproliferative properties of our SP antagonists also allow their use in the prevention of glial scarring (thus facilitating nerve regeneration). The action of our antagonists on neurotransmission allow their use as nonopiate analgesics. Their use as nonopiate analgesics can permit the restoration of opiate response. Our antagonists are also useful as antisecretory agents, acting e.g., on the salivary glands or on the pancreas.

In the generic formulas given above, when any of R₁, R₂, R₁₀-R₁₂, R₅₁, R₅₂, or R₆₆-R₆₈, is an aromatic, lipophilic group, the in vivo activity can be long lasting, and delivery of our compounds to the target tissue can be facilitated.

The identifying group of an α-amino acid is the atom or group of atoms, other than the α-carbonyl carbon atom, the α-amino nitrogen atom, or the H atom, bound to the asymmetric α-carbon atom. To illustrate by examples, the identifying group of alanine is CH₃, the identifying group of valine is (CH₃)₂CH, the identifying group of lysine is H₃N⁺(CH₂)₄ and the identifying group of phenylalanine is (C₆H₅)CH₂. The identifying group of a β- or γ-amino acid is the analagous atom or group of atoms bound to respectively, the β- or the γ-carbon atom. Where the identifying group of an amino acid is not specified it may be α, β, or γ.

Other features and advantages of the invention will be apparent from the following description of the preferred embodiments thereof.

We first briefly describe the drawings.

In the drawings:

Fig. 1 is a pair of graphs illustrating the effect of pseudopeptides of spantide, D-Arg-Pro-Lys-Pro-Gln-Gln-D-Trp-Phe-D-Trp-Leu-Leu-NH₂, (left panel) and of SP, Arg-Pro-Lys-Pro-Gln-Gln-Phe-Phe-Gly-Leu-Met-NH₂, (right panel) on SP- stimulated amylase release from pancreatic acini.

Fig. 2 is a graph showing the effect of Arg-Pro-Lys-Pro-Gln-Gln-Phe-Phe-Gly-Leu^{L}[CH₂-NH]Leu-NH₂ on SP-stimulated amylase release from pancreatic ancini.

Fig. 3 is a pair of graphs illustrating the ability of various SP and spantide pseudopeptides to inhibit binding of ¹²⁵I-BH-substance P to pancreatic ancini.

Fig. 4 is a graph showing the ability of sP and spantide pseudopeptides to inhibit binding of ¹²⁵I-[Tyr⁴] bombesin to pancreatic acini.

We now describe the structure, synthesis, and use of preferred embodiments of our SP analogs.

Our peptides all have modifications, e.g., a non-peptide bond in at least one of the indicated positions in which the carbon atom participating in the bond between two residues is reduced from a carbonyl carbon to a methylene carbon. The peptide bond reduction method which yields this non-peptide bond is described in Coy et al., U.S. patent application, Serial No. 879,348 (EP-A-0 257 742), assigned to the same assignee as the present application, hereby incorporated by reference.

Our peptides can be provided in the form of pharmaceutically acceptable salts. Examples of preferred salts are those with therapeutically acceptable organic acids, e.g., acetic, lactic, maleic, citric, malic, ascorbic, succinic, benzoic, salicylic, methanesulfonic, toluenesulfonic, or pamoic acid, as well as polymeric acids such as tannic acid or carboxymethyl cellulose, and salts with inorganic acids such as the hydrohalic acids, e.g., hydrochloric acid, sulfuric acid, or phosphoric acid.

### Synthesis of Substance P Analogs

The synthesis of the substance P antagonist Arg-Pro-Lys-Pro-Gln-Gln-Phe-Phe-Gly-Leu^{L}[CH₂NH]Leu-NH₂ follows. Other substance P analogs can be prepared by making the appropriate modifications of the following synthetic method.

The first step is the preparation of the intermediate Arg-Pro-Lys-Pro-Gln-Gln-Phe-Phe-Gly-Leu^{L}[CH₂-NH]Leu-benzhydrylamine resin, as follows.

Benzhydrylamine-polystyrene resin (Vega Biochemicals, Inc.) (0.97 g, 0.5 mmole) in the chloride ion form is placed in the reaction vessel of a Beckman 990B peptide synthesizer programmed to perform the following reaction cycle: (a) methylene chloride; (b) 33% trifluoroacetic acid (TFA) in methylene chloride (2 times for 1 and 25 min. each); (c) methylene chloride; (d) ethanol; (e) methylene chloride; and (f) 10% triethylamine in chloroform.

The neutralized resin is stirred with alpha-t-butoxycarbonyl(Boc)-leucine and diisopropylcarbodiimide (1.5 mmole each) in methylene chloride for 1 hour, and the resulting amino acid resin is then cycled through steps (a) to (f) in the above wash program. Boc-leucine aldehyde (1.25 mmoles), prepared by the method of Fehrentz and Castro, Synthesis, p. 676 (1983), is dissolved in 5 ml of dry dimethylformamide (DMF) and added to the resin TFA salt suspension followed by the addition of 100 mg (2 mmoles) of sodium cyanoborohydride (Sasaki and Coy, Peptides 8:119-121 (1987); Coy et al., id.). After stirring for 1 hour, the resin mixture is found to be negative to ninhydrin reaction (1 min.), indicating complete derivatization of the free amino group.

The following amino acids (1.5 mmole) are then coupled successively in the presence diisopropylcarbodiimide (1.5 mmole), and the resulting amino acid resin is cycled through washing/deblocking steps (a) to (f) in the same procedure as above: Boc-Gly (Boc-Gly is coupled as a 6M excess of the p-nitrophenylester), Boc-Phe, Boc-Phe, Boc-Gln, Boc-Gln, (Boc-Gln is coupled as a 6 M excess of the p-nitrophenylester), Boc-Pro, Boc-Lys, Boc-Pro, and Boc-Arg. The completed resin is then washed with methanol and air dried.

The resin described above (1.6 g, 0.5 mmole) is mixed with anisole (5 ml) and anhydrous hydrogen fluoride (35 ml) at 0°C and stirred for 45 min. Excess hydrogen fluoride is evaporated rapidly under a stream of dry nitrogen, and free peptide is precipitated and washed with ether. The crude peptide is dissolved in a minimum volume of 2 M acetic acid and purified on a column (2.5 x 90 cm) of Sephadex G-25 which is eluted with 2 M acetic acid, followed by preparative medium pressure chromatography on a column (1.5 x 45 cm) of Vydac C₁₈ silica (10-15 µm) which is eluted with linear gradients of acetonitrile in 0.1% trifluoroacetic acid using an Eldex Chromatrol gradient controller (flow rate 1 ml/min). Where necessary, analogues are further purified by re-chromatography on the same column with slight modifications to the gradient conditions when necessary. Homogeneity of the peptides was assessed by thin layer chromatography and analytical reverse-phase high pressure liquid chromatography, and purity was 97% or higher. Amino acid analysis gave the expected amino acid ratios. The presence of the reduced peptide bond was demonstrated by fast atom bombardment mass spectrometry. Each of the analogues gave good recovery of the molecular ion corresponding to the calculated molecular mass.

Other compounds can be prepared as above and tested for effectiveness as agonists or antagonists in the following test program.

### Phase 1- Amylase Release From Pancreatic Acini

SP stimulates the release of amylase in pancreatic acini. The stimulation or inhibition of release of amylase by pancreatic acini is used as a measure of, respectively, the agonist or antagonist activity of a peptide. Dispersed acini from the pancreas of one animal are suspended in 150 ml of standard incubation solution. Amylase release is measured as described previously (Gardner et al. (1977) J. Physiol. 270:439). Amylase activity is determined by the methods of Ceska et al. (Ceska et al. (1969) Clin. Chim. Acta. 26:437 and Ceska et al. (1969) Clin. Chim. Acta. 26:445) using the Phadebas reagent. Amylase release is calculated as the percentage of the amylase activity in the acini at the beginning of the incubation that was released into the extracellular medium during the incubation.

### Phase 2 - Competitive Inhibition of ¹²⁵ I-Bolton-Hunter-SP Binding

Binding of ¹²⁵I-Bolton-Hunter-SP (¹²⁵I-BH-SP) to dispersed pancreatic acini is measured as described previously (Jensen et al. (1984) Biochem. Biophys. Acta. 804:181 and Jensen et al. (1988) Am. J. Physiol. 254:G883). Incubations contain 0.125 nM ¹²⁵I-BH-SP and 0.1% bacitracin in standard incubation buffer and were for 30 min at 37°C. Nonsaturable binding of ¹²⁵I-BH-SP is the amount of radioactivity associated with the acini when the incubation contains 0.125 nM ¹²⁵I-BH-SP plus 1 µM unlabeled SP. All values given are for saturable binding, i.e., binding measured with ¹²⁵I-BH-SP alone (total binding). In all experiments nonsaturable binding was < 30% of total binding.

### Phase 3- Competitive Inhibition of ¹²⁵I-[Tyr⁴] Bombesin Binding

¹²⁵I-[Tyr⁴] Bombesin (2200 Ci/mmol) is prepared using a modification of the method described previously (Jensen et al. (1978) Proc. Natl. Acad. Sci. USA 75:6139). Iodo-Gen (mg) is dissolved in 5 ml of chloroform and 5 µl of this solution (1 µg Iodo-Gen) is transferred to a vial under a stream of nitrogen. To this vial 50 µl of KH₂PO₄ (pH 7.4), 6 µg [Tyr⁴]bombesin in 5 µl water and 1 mCi Na¹²⁵I is added, mixed and incubated for 6 min at 4°C, at which time the iodination mixture is added to a vial of 1 M dithiothreitol and incubated at 80°C for 60 min. The iodination mixture is then loaded onto a Sep-Pak cartridge and eluted with 0.25 M tetraethylammonium phosphate (TEAP) followed by 50% (vol/vol) acetonitrile-0.25 M TEAP. ¹²⁵I-[Tyr⁴]bombesin is purified using reverse-phase high-performance liquid chromatography (HPCL) and eluted.

### Results of Assays of Test Peptides

A number of analogues of substance P, or of the substance P antagonist spantide, each containing a non-peptide bond, can be synthesized and tested in one or more of the assays described in Phase 1 - Phase 3 above. The structure of substance P is Arg-Pro-Lys-Pro-Gln-Gln-Phe-Phe-Gly-Leu-Met-NH₂. Spantide is an analog of SP. The structure of spantide is D-Arg-Pro-Lys-Pro-Gln-Gln-D-Trp-Phe-D-Trp-Leu-Leu-NH₂. Stimulation or inhibition of the release of amylase from dispersed pancreatic acini was used as an assay for SP agonist activity or SP antagonist activity respectively. At a concentration of 10 µM, 9 of 10 SP and spantide derived pseudopeptides failed to stimulate amylase release when present alone (Table 2). One peptide, Arg-Pro-Lys-Pro-Gln-Gln-Phe-Phe- Gly^{L}[CH₂-NH]Leu-Leu-NH₂, (10 µM) had agonist activity causing a 3-fold increase in amylase release (Table 2). Each of the 9 pseudopeptides without agonist activity was examined for activity as a SP antagonist. At a concentration of 10µM each of the spantide derived pseudopeptide analogues inhibited 1 nM-SP-stimulated amylase release. (Table 2) Three SP pseudopeptides, Arg-Pro-Lys-Pro-Gln-Gln-Phe-Phe-Gly-Leu^{L}[CH₂-NH]Leu-NH₂, Arg-Pro-Lys-Pro-Gln-Gln-Phe-Phe^{L}[CH₂-NH]Gly-Leu-Leu-NH₂, and Arg-Pro-Lys-Pro-Gln-Gln^{L}[CH₂-NH]Phe-Phe-Gly-Leu-Leu-NH₂ caused inhibition (Table 2).

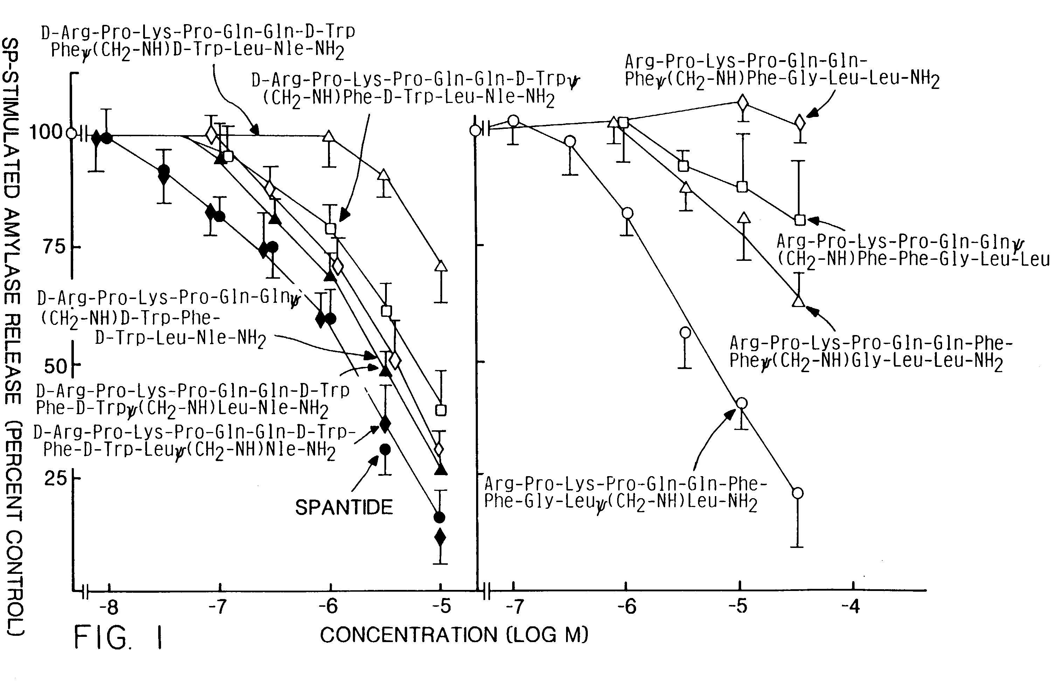

The relative abilities of each peptide to inhibit SP-stimulated amylase release was determined by the effect of peptide dose on inhibition. Dose-inhibition studies were carried out for each of the 9 pseudopeptides using a concentration of SP (1nM) that causes half-maximal stimulation. (Fig. 1). Results for the 5 spantide derived pseudopeptides are shown in Fig. 1, left panel. D-Arg-Pro-Lys-Pro-Gln-Gln-D-Trp-Phe-D-Trp-Leu^{L}[CH₂-NH]Nle-NH₂ was equipotent to spantide, causing detectable inhibition at 0.03 µM and half-maximal inhibition at 1.8 µM (Table 3). D-Arg-Pro-Lys- Pro-Gln-Gln-D-Trp-Phe-D-Trp^{L}[CH₂-NH]Leu-Nle-NH₂ was 2-fold less potent (IC₅₀3.5 µM, Table 3) than spantide. D-Arg-Pro-Lys-Pro-Gln-Gln^{L}[CH₂-NH]D-Trp-Phe-D-Trp-Leu-Nle-NH₂ was 2.6-times (IC₅₀ 4.7 µM) less potent than spantide. D-Arg-Pro-Lys-Pro-Gln-Gln-D-Trp^{L}[CH₂-NH]Phe-D-Trp-Leu-Nle-NH₂ was 3.5-times (IC₅₀, 6.4-µM) less potent and D-Arg-Pro-Lys-Pro-Gln-Gln-D-Trp-Phe^{L}[CH₂-NH]D-Trp-Leu-Nle-NH₂ was 17-times (IC₅₀, 30 µM) less potent than spantide (Table 3).

Results for the SP pseudopeptide analogues are shown in Fig. 1, right panel. Arg-Pro-Lys-Pro-Gln-Gln-Phe-Phe-Gly-Leu^{L}[CH₂-NH]Leu-NH₂ was the most potent SP derivative causing detectible inhibition at 0.3 µM and half-maximal inhibition at 7.1 µM (Table 3, right). Arg-Pro-Lys-Pro-Gln-Gln-Phe-Phe^{L}[CH₂-NH]Gly-Leu-Leu-NH₂ and Arg-Pro-Lys-Pro-Gln-Gln^{L}[CH₂-NH]Phe-Phe-Gly-Leu-Leu-NH₂ were less effective, causing detectable inhibition at 10 µM. They are, respectively, 7-fold and 46-fold less potent than Arg-Pro-Lys-Pro-Gln-Gln-Phe-Phe- Gly-Leu^{L}[CH₂-NH]Leu-NH₂ (Table 3, right). Arg-Pro-Lys-Pro-Gln-Gln-Phe^{L}[CH₂-NH]Phe-Gly-Leu-Leu-NH₂ exhibited no inhibitory activity at concentrations as high as 30 µM (Fig. 1, right). The most potent SP derived pseudopeptide antagonist was Arg-Pro-Lys-Pro-Gln-Gln-Phe-Phe- Gly-LeuL[CH2-NH]Leu-NH2 (Fig. 1, Table 2).

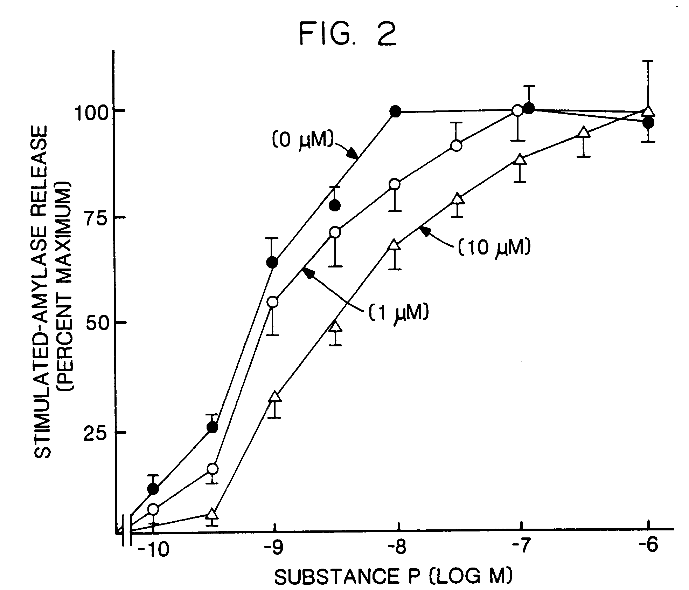

The inhibitory effects of the most potent SP derived pseudopeptide antagonist, Arg-Pro-Lys-Pro-Gln-Gln-Phe-Phe-Gly-Leu^{L}[CH₂-NH]Leu-NH₂, are shown in Fig. 2. Acini were incubated with increasing concentrations of SP. Amylase release was detectible with 0.1 nM SP, was half-maximal with 1 nM SP2, and was maximal with 10 nM (Fig. 2). Addition of Arg-Pro-Lys-Pro-Gln-Gln-Phe-Phe-Gly-Leu^{L}[CH₂-NH]Leu-NH₂ caused a parallel rightward shift in the dose-response curve for SP-stimulated amylase release. The shift was proportional to the concentration of Arg-Pro-Lys-Pro-Gln-Gln-Phe-Phe-Gly-Leu^{L}[CH₂-NH]Leu-NH₂ added but there was no change in the maximal response (Fig. 2). D-Arg-Pro-Lys-Pro-Gln-Gln-D-Trp- Phe-D-Trp-Leu^{L}[CH₂-NH]Nle-NH₂ gave similar results (data not shown).

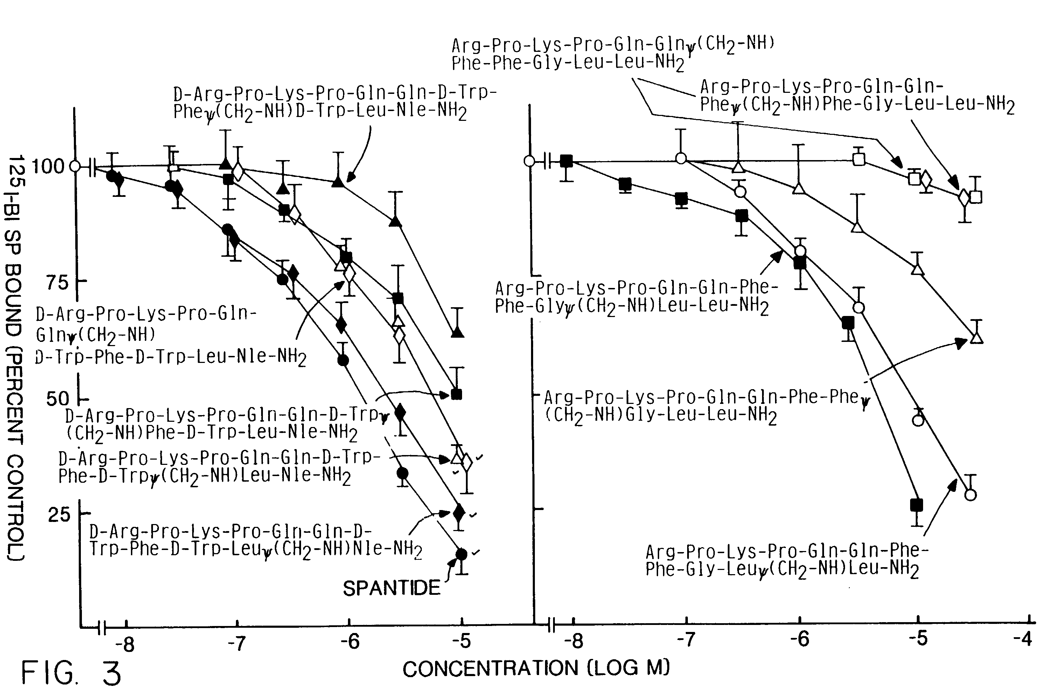

The interaction of SP and spantide derived analogues with the SP receptors of pancreatic acini was measured by the ability of a peptide to inhibit the binding of ¹²⁵I-BH-SP to acini. (See Table 3 and Fig. 3.)

The spantide derived pseudopeptides show a range of potencies. D-Arg-Pro-Lys-Pro-Gln-Gln-D-Trp-Phe-D-Trp-Leu^{L} [CH₂NH]Nle-NH₂ is roughly equivalent in potency to spantide causing detectible inhibition at 0.03 µM and half-maximal inhibition at 2.2 µM (Fig. 3, left, Table 3). D-Arg-Pro-Lys-Pro-Gln-Gln-D-Trp-Phe-D-Trp^{L}[CH₂-NH]Leu-Nle-NH₂ and D-Arg-Pro-Lys-Pro-Gln-Gln^{L}[CH₂-NH]D-Trp-Phe-D-Trp-Leu-Nle-NH₂ are 2-fold less potent than spantide. D-Arg-Pro-Lys-Pro-Gln-Gln-D-Trp^{L}[CH₂-NH]Phe-D-Trp-Leu-Nle-NH₂ was 3-times (Kᵢ, 6.3 µM) less potent than spantide. D-Arg-Pro-Lys-Pro-Gln-Gln-D-Trp-Phe^{L}[CH₂-NH]D-Trp-Leu-Nle-NH₂ was 7-times (Kᵢ, 14.7 µM) less potent than spantide (Fig. 3, Table 3).

The SP derived pseudopeptides also show a range of potencies. Arg-Pro-Lys-Pro-Gln-Gln-Phe-Phe-Gly-Leu^{L}[CH₂-NH]Leu-NH₂ causes detectable inhibition at 0.1 µM, and half-maximal inhibition at 3 µM. Arg-Pro-Lys-Pro-Gln-Gln-Phe-Phe-Gly^{L}[CH₂-NH]Leu-Leu-NH₂ is 1.5-fold lower in potency, Arg-Pro-Lys-Pro-Gln-Gln-Phe-Phe^{L}[CH₂-NH]Gly-Leu-Leu-NH₂ is 20-times less potent, and Arg-Pro-Lys-Pro-Gln-Gln-Phe^{L}[CH₂-NH]Phe-Gly-Leu-Leu-NH₂ and Arg-Pro-Lys-Pro-Gln-Gln^{L}[CH₂-NH]Phe-Phe-Gly-Leu-Leu-NH₂ are more than 62-times less potent than Arg-Pro-Lys-Pro-Gln-Gln-Phe-Phe-Gly-Leu^{L}[CH₂-NH]Leu-NH₂.

Unlike previously studied SP analogs our peptides tested appear to be specific to the SP receptor.
Arg-Pro-Lys-Pro-Gln-Gln-Phe-Phe-Gly-Leu^{L}[CH₂-NH]Leu-NH₂ was tested for the ability to inhibit amylase release produced by various pancreatic secretagogues (Table 4). Arg-Pro-Lys-Pro-Gln-Gln-Phe-Phe-Gly-Leu^{L}[CH₂-NH]Leu-NH₂ (20 µM) inhibited amylase release stimulated by SP, but did not alter bombesin, CCK-8, carbachol, VIP, secretin, CGRP A23187 or TPA stimulated amylase release.

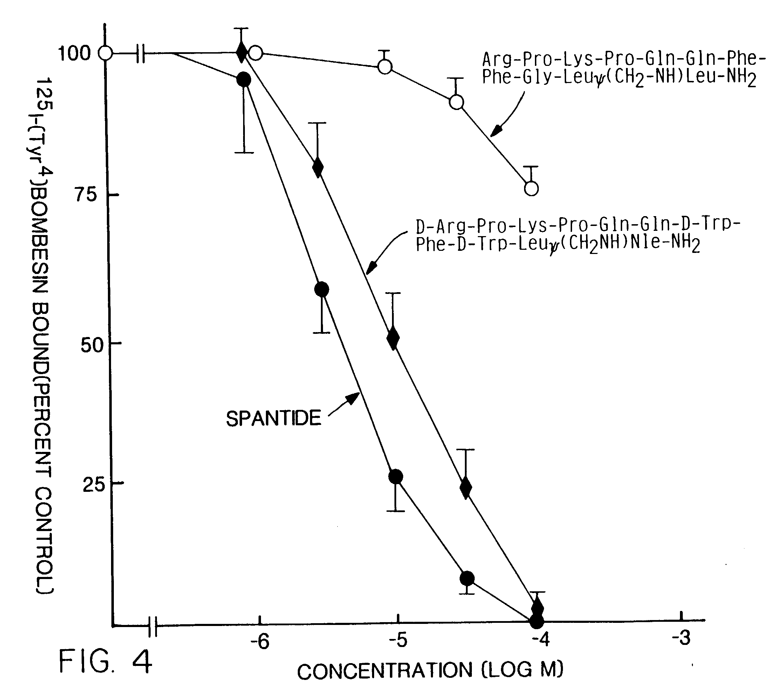

The most potent receptor antagonists were tested for their ability to inhibit the binding of ¹²⁵I-[Tyr⁴] bombesin to the bombesin receptors of pancreatic acini. Spantide inhibited binding of ¹²⁵I-[Tyr⁴] bombesin as reported previously (Jensen et al. (1988) Am. J. Physiol. 254:G883) causing half-maximal inhibition at 3 ± 1 µM and complete inhibition at 100 µM (Fig. 4). D-Arg-Pro-Lys-Pro-Gln-Gln-D-Trp-Phe-D-Trp-Leu^{L}[CH₂-NH]Nle-NH₂ inhibited ¹²⁵I-[Tyr⁴]bombesin binding but was 3-fold less potent than spantide causing half-maximal inhibition at 10 µM (p < 0.05 compared to spantide) (Fig. 4). Arg-Pro-Lys-Pro-Gln-Gln-Phe-Phe-Gly-Leu^{L}[CH₂-NH]Leu-NH₂ did not cause detectable inhibition until concentrations above 30 µM and had a calculated Kᵢ of 300 ± 20 µM. Spantide and D-Arg-Pro-Lys-Pro-Gln-Gln-D-Trp-Phe-D- Trp-Leu^{L}[CH₂-NH]Nle-NH₂ had a 3- to 10-fold lower affinity for inhibiting ¹²⁵I[Tyr⁴]bombesin binding as compared to ¹²⁵I-BH-SP.
The ability of Arg-Pro-Lys-Pro-Gln-Gln-Phe-Phe-Gly-Leu^{L} [CH₂-NH]Leu-NH₂ to inhibit binding of ¹²⁵I-[Tyr⁴] bombesin was 70-fold lower than its ability to inhibit binding of ¹²⁵I-BH-SP.

Our SP analogs may be administered to a mammal, particularly a human, in one of the traditional modes (e.g., orally, parenterally, transdermally, or transmucosally), in a sustained release formulation using a biodegradable biocompatible polymer, or by on-site delivery using micelles, gels and liposomes.

The peptides can be administered to a human patient in a dosage of 0.5 µg/kg/day to 5 mg/kg/day.

**Table 1.**

| The five carboxyl-terminal residues of a variety of bioactive peptides. | |
|---|---|
| Peptide | Carboxyl Terminal Sequence |
| Bombesin | -Val-Gly-His-Leu-Met-NH₂ |
| Neuromedin-B | -Thr-Gly-His-Phe-Met-NH₂ |
| Neuromedin-C | -Val-Gly-His-Leu-Met-NH₂ |
| Litorin | -Val-Gly-His-Phe-Met-NH₂ |
| Neurokinin-A | -Phe-Val-Gly-Leu-Leu-NH₂ |
| Neurokinin-B | -Phe-Val-Gly-Leu-Met-NH₂ |
| Substance P | -Phe-Phe-Gly-Leu-Met-NH₂ |
| Prototypical Tachykinin Sequence | -Phe-X-Gly-Leu-Met-NH₂ (where X = a branched aliphatic or aromatic amino acid residue) |

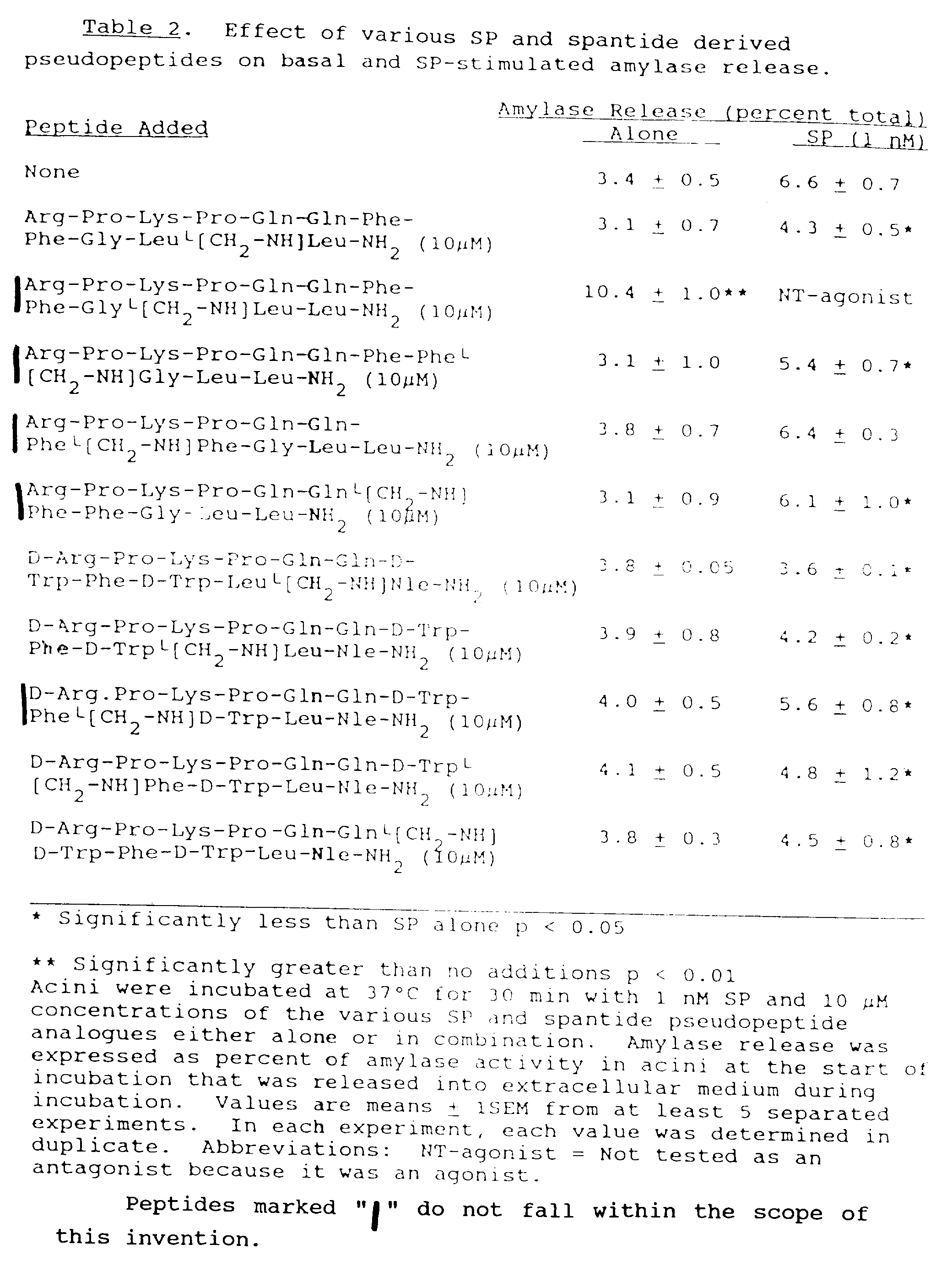

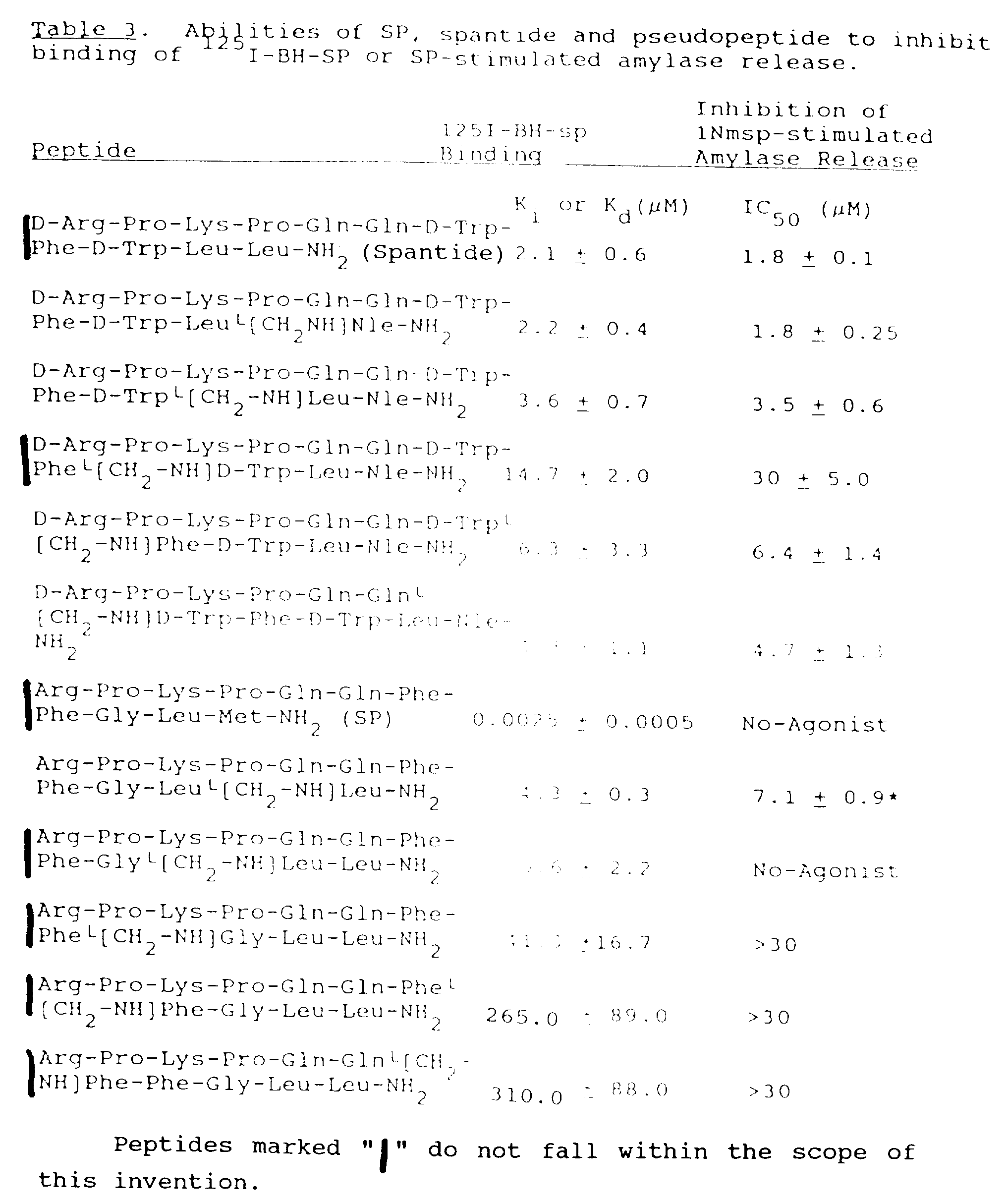

Values are means ± 1SEM Kd values for SP are obtained from Scatchard analysis of ¹²⁵I-labeled SP binding studies K₁ values for agonist or antagonists from studies of binding ¹²⁵I-BH-SP were obtained according to the equation: Kᵢ = (R/1-R) (SB/S+A) where R is the observed saturable binding of ¹²⁵I-BH-SP in the presence of antagonist (B) expressed as a fraction of that obtained when B is not present; A is the concentration of ¹²⁵I-BH-SP (0.125 nM). B is the concentration of antagonist, S is the K_{d} of SP determined by Scatchard analysis. No-agonist = peptide not tested for inhibition activity because agonist activity when present alone.

**Table 4.**

| Ability of Arg-Pro-Lys-Pro-Gln-Gln-Phe-Phe-Gly-Leu^{L}[CH₂-NH]Leu-NH₂ to affect amylase release stimulated by various secretagogues. | | |
|---|---|---|
| | Amylase Release (percent total) | |
| Secretagogue | Alone | Leu¹¹, ^{L}10-11-SP (20 µM) |
| None | 2.7 ± 0.4 | 2.3 ± 0.3 |
| Substance P (1 nM) | 8.7 ± 1.8 | 4.3 ± 0.5* |
| CCK-8 (0.1 nM) | 19.7 ± 4.2 | 21.6 ± 4.9 |
| Bombesin (0.3 nM) | 14.8 ± 4.1 | 14.3 ± 3.1 |
| Carbachol (10 µM) | 22.7 ± 4.1 | 20.7 ± 4.3 |
| VIP (0.3 nM) | 20.5 ± 3.5 | 18.9 ± 4.2 |
| Secretin (0.1 µM) | 18.9 ± 3.6 | 18.8 ± 4.1 |
| CGRP (0.1 µM) | 12.1 ± 3.3 | 12.0 ± 3.9 |
| A23187 (0.1 µM) | 9.9 ± 1.5 | 11.6 ± 1.6 |
| TPA (0.1 µM) | 32.2 ± 5.7 | 30.2 ± 5.6 |

| | | |
|---|---|---|
| *Significantly less than secretagogue alone p < 0.001 Acini were incubated for 30 min at 37°C with various pancreatic secretagogues alone or with Arg-Pro-Lys-Pro-Gln-Gln-Phe-Phe-Gly-Leu^{L}[CH₂-NH]LeuNH₂. In each experiment, each value was determined in duplicate, and results give means ± 1SEM from at least 4 separated experiments. Abbreviations: CCK-8, COOH-terminal octapeptide of cholecystokinin; VIP, vasoactive intestinal peptide, respectively; CGRP, calcitonin gene-related peptide; A23187 and TPA, 1,2-o-tetradecanoylphorbol-1,3-acetate. | | |

## Claims

1. A substance P analog of the formula: 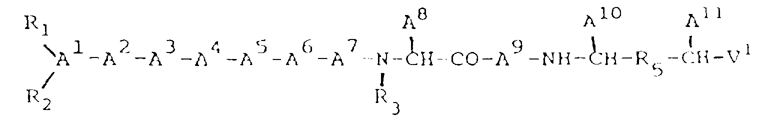 wherein
A¹ = the D or L isomer of Arg or Lys:
A² = the D or L isomer of Pro;
A³ = the D or L isomer of Lys;
A⁴ = the D or isomer of Pro;
A⁵ = the D or L isomer of Gln;
A⁶ = the D or L isomer of Gln;
A⁷ = the D or L isomer of Phe, Trp, β-Nal, o-X-Phe in which X is F, Cl, Br, NO₂, OH, or CH₃, or of p-X-Phe in which X is F, Cl, Br, NO₂, OH, or CH₃;
A⁸ = the identifying group of the D or L isomer of Trp, β-Nal, Phe, o-X-Phe in which X is F, Cl, Br, NO₂, OH or CH₃, or of p-x-Phe in which X is F, Cl, Br, NO₂, OH, or CH₃;
A⁹ = Gly or the D or L isomer of Trp, β-Nal, Phe, o X Phe in which X is F, Cl, Br, NO₂, OH, or CH₃, or of p-X-Phe in which X is F, Cl, Br, NO₂, OH, or CH₃;
A¹⁰ = the identifying group of the D or L isomer of Leu, Nle, Ala, Val or of Ile;
A¹¹ = the identifying group of the D or L isomer of Leu, Nle, Ala, Val or of Ile;
R₃ is H or C₁₋₁₂ alkyl; R₅ is CH₂-NH; and V¹ is 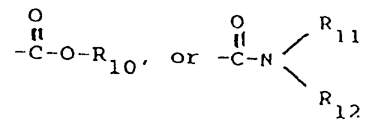 in which each R₁₀, R₁₁, and R₁₂ independently, is H, C₁₋₁₂ alkyl, C₇₋₁₀ phenylalkyl, or C₁₂₋₂₀ naphthylalkyl; and R₁ and R₂, which are bonded to the nitrogen of the α-amino group at the N-terminal amino acid are, independently, H, C₁₋₁₂ alkyl, C₇₋₁₀ phenylalkyl, or COE₁₄ with E₁₄ being C₁₋₂₀ alkyl, C₃₋₂₀ alkenyl, C₃₋₂₀ alkynyl, phenyl, naphthyl, or C₇₋₁₀ phenylalkyl, provided that when one of R₁ or R₂ is COE₁₄, the other must be a hydrogen;
or a pharmaceutically acceptable salt thereof.

2. A substance P analog of the formula: 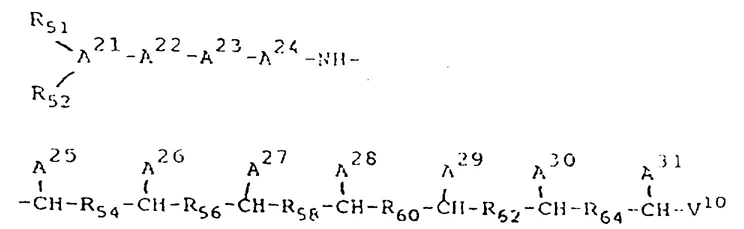 wherein
A²¹ = the D or L isomer of Arg or Lys;
A²² = the D or L isomer of Pro;
A²³ = the D or L isomer of Lys;
A²⁴ = the D or L isomer of Pro;
A²⁵ = the identifying group of the D or L isomer of Gln;
A²⁶ = the identifying group of the D or L isomer of Gln;
A²⁷ = the identifying group of D-Trp; or the identifying group of the D or L isomer of Phe;
A²⁸ = the identifying group of the D or L isomer of any one of the amino acids β-Nal, Phe, o-X-Phe in which X is F, Cl, Br, NO₂, OH, or CH₃, or p-X-Phe in which X is F, Cl, Br, NO₂, OH, or CH₃;
A²⁹ = the identifying group of the amino acid D-Trp; or the identifying group of the D or L isomer of Phe;
A³⁰ = the identifying group of the D or L isomer of any one of the amino acids Leu, Nle, Ala, Val or Ile;
A³¹ = the identifying group of the D or L isomer of any one of the amino acids Leu, Nle, Ala, Val or Ile;
R₅₄ is CO-NH or CO-NCH₃; each R₅₆, R₆₂, and R₆₄,
independently, is any of CO-NH or CH₂-NH, R₅₈ is CO-NR₆₉ in which R₆₉ is H or C₁₋₁₂ alkyl, or CH₂-NH, R₆₀ is CO-NH; and V¹⁰ is 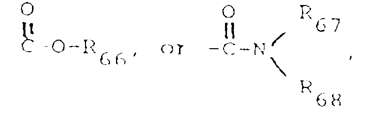 in which each R₆₆, R₆₇, and R₆₈ independently, is H, C₁₋₁₂ alkyl, C₇₋₁₀ phenylalkyl, or C₁₂₋₂₀ naphthylalkyl; and R₅₁ and R₅₂, which are bonded to the nitrogen of the α-amino group at the N-terminal amino acid are, independently, H, C₁₋₁₂ alkyl, C₇₋₁₀ phenylalkyl or COE₂₄ with E₂₄ being C₁-C₂₀ alkyl, C₃₋₂₀ alkenyl, C₃₋₂₀ alkenyl, phenyl, naphthyl, or C₇₋₁₀ phenylalkyl provided that when one of R₅₁ or R₅₂ is COE₂₄, the other is H; with the proviso that at least one of R₅₆, R₅₈, R₆₂, or R₆₄ is CH₂NH; or a pharmaceutically acceptable salt thereof.

3. A substance P analog according to Claim 1 of the formula: H-Arg-Pro-Lys-Pro-Gln-Gln-Phe-Phe-Gly-Leu^{L}[CH₂-NH]Leu-NH₂; or a pharmaceutically acceptable salt thereof.

4. A substance P analog according to Claim 2 of the formula: H-D-Arg-Pro-Lys-Pro-Gln-Gln-D-Trp-Phe-D-Trp^{L}[CH₂-NH]Leu-Nle-NH₂; or a pharmaceutically acceptable salt thereof.

5. A substance P analog according to Claim 2 of the formula: H-D-Arg-Pro-Lys-pro-Gln-Gln-D-Trp-Phe-D-Trp-Leu^{L}[CH₂-NH]Nle-NH₂; or a pharmaceutically acceptable salt thereof.

6. A substance P analog according to Claim 2 of the formula: H-D-Arg-Pro-Lys-Pro-Gln-Gln-D-Trp^{L}[CH₂-NH]Phe-D-Trp-Leu-Nle-NH₂; or a pharmaceutically acceptable salt thereof.

7. A substance P analog according to Claim 2 of the formula: H-D-Arg-Pro-Lys-Pro-Gln-Gln-^{L}[CH₂-NH]D-Trp-Phe-D-Trp-Leu-Nle-NH₂; or a pharmaceutically acceptable salt thereof.

8. A substance P analog according to Claim 1, wherein
A¹ = the D or L isomer of Arg;
A² = Pro;
A³ = Lys;
A⁴ = Pro;
A⁵ = Gln;
A⁶ = Gln;
A⁷ = Phe or D-Trp;
A⁸ = the identifying group of Phe;
A⁹ = Gly or D-Trp;
A¹⁰ = the identifying group of Leu; and
A¹¹ = the identifying group of Leu or of Nle.

9. A substance P analog according to Claim 2, wherein
A²⁹ = the identifying group of D-Trp.

10. A substance P analog according to Claim 2, wherein
A²¹ = the D or L isomer of Arg;
A²² = Pro;
A²³ = Lys;
A²⁴ = Pro;
A²⁵ = the identifying group of Gln;
A²⁶ = the identifying group of Gln;
A²⁷ = the identifying group of Phe or of D-Trp;
A²⁸ = the identifying group of Phe;
A²⁹ = the identifying group of D-Trp;
A³⁰ = the identifying roup of Leu; and
A³¹ = the identifying group of Leu or of Nle.

## Patentansprüche

1. Analoges der Substanz P mit der Formel: 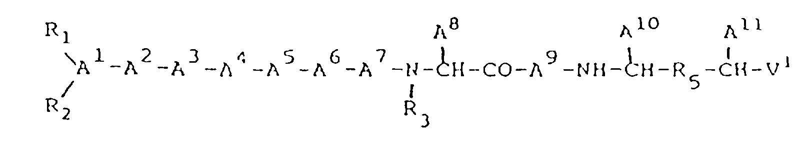 worin
A¹ = das D- oder L-lsomere von Arg oder Lys;
A² = das D- oder L-lsomere von Pro;
A³ = das D- oder L-lsomere von Lys;
A⁴ = das D- oder L-lsomere von Pro;
A⁵ = das D- oder L-lsomere von Gln;
A⁶ = das D- oder L-lsomere von Gln;
A⁷ = das D- oder L-lsomere von Phe, Trp, β-Nal, o-X-Phe, wobei X = F, Cl, Br, NO₂, OH oder CH₃,
oder von p-X-Phe, wobei X = F, Cl, Br, NO₂, OH oder CH₃,
A⁸ = die charakterisierende Gruppe des D- oder L-lsomeren von Trp, β-Nal, Phe, o-X-Phe, wobei X = F, Cl, Br, NO₂, OH oder CH₃, oder von p-X-Phe, wobei X = F, Cl, Br, NO₂, OH oder CH₃;
A⁹ = Gly oder das D- oder L-Isomere von Trp, β-Nal, Phe oder o-X-Phe, wobei X = F, Cl, Br, NO₂, OH oder CH₃, oder von p-X-Phe, wobei X = F, Cl, Br, NO₂, OH oder CH₃;
A¹⁰ = die charakterisierende Gruppe des D- oder L-lsomeren von Leu, Nle, Ala, Val oder Ile;
A¹¹ = die charakterisierende Gruppe des D- oder L-lsomeren von Leu, Nle, Ala, Val oder Ile; worin
R₃ H oder C₁₋₁₂Alkyl, R₅ CH₂-NH und V¹ 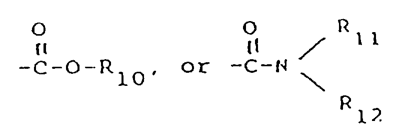 bedeuten, wobei R₁₀, R₁₁ und R₁₂ jeweils unabhängig voneinander für H, C₁₋₁₂Alkyl, C₇₋₁₀Phenylalkyl oder C₁₂₋₂₀Naphthypatkyl; R₁ und R₂, die an den Stickstoff der α-Aminogruppe an der N-terminalen Aminosäure gebunden sind, unabhängig voneinander für H, C₁₋₁₂Alkyl, C₇₋₁₀Phenylalkyl oder COE₁₄ stehen, wobei E₁₄ C₁₋₂₀Alkyl, C₃₋₂₀Alkenyl, C₃₋₂₀Alkinyl, Phenyl, Naphthyl oder C₇₋₁₀Phenylalkyl bedeuten, vorausgesetzt, daß es sich bei dem jeweils anderen Rest um Wasserstoff handeln muß, wenn einer der Reste R₁ oder R₂ COE₁₄ ist; oder ein pharmazeutisch nutzbares Salz davon.

2. Analoges der Substanz P mit der Formel 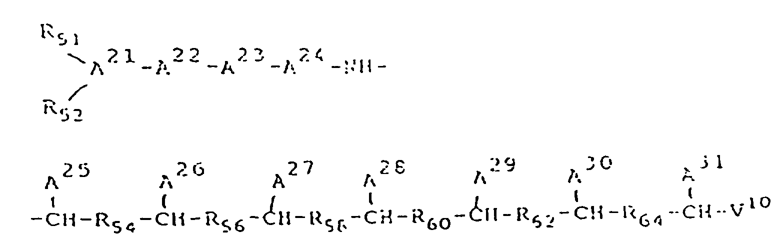 worin
A²¹ = das D- oder L-Isomere von Arg oder Lys;
A²² = das D- oder L-Isomere von Pro;
A²³ = das D- oder L-Isomere von Lys;
A²⁴ = das D- oder L-Isomere von Pro;
A²⁵ = die charakterisierende Gruppe des D- oder L-lsomeren von Gln;
A²⁶ = die charakterisierende Gruppe des D- oder L-lsomeren von Gln;
A²⁷ = die charakterisierende Gruppe des D- oder L-lsomeren von D-Trp oder die charakterisierende Gruppe des D- oder L-lsomeren von Phe;
A²⁸ = die charakterisierende Gruppe des D- oder L-lsomeren einer der Aminosäuren β-Nal, Phe oder o-X-Phe, wobei X = F, Cl, Br, NO₂, OH oder CH₃, oder von p-X-Phe, wobei X = F, Cl, Br, NO₂, OH oder CH₃;
A²⁹ = die charakterisierende Gruppe der Aminosäure D-Trp oder die charakterisierende Gruppe des D- oder L-lsomeren von Phe;
A³⁰ = die charakterisierende Gruppe des D- oder L-lsomeren einer der Aminosäuren Leu, Nle, Ala, Val oder Ile;
A³¹ = die charakterisierende Gruppe des D- oder L-lsomeren einer der Aminosäuren Leu, Nle, Ala, Val oder Ile;
R₅₄ CO-NH oder CO-NCH₃
R₅₆, R₆₂ und R₆₄ jeweils unabhängig voneinander CO-NH oder CH₂-NH und R₅₈ CO-NR₆₉ bedeuten, worin R₆₉ für H, C₁₋₁₂Alkyl oder CH₂-NH steht, R₆₀ CO-NH und V¹⁰ 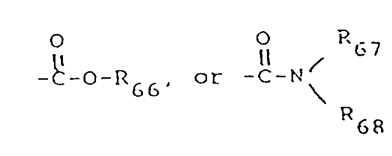 bedeuten,
worin R₆₆, R₆₇ und R₆₈ jeweils unabhängig voneinander für H, C₁₋₁₂Alkyl, C₇₋₁₀Phenylalkyl, C₁₂₋₂₀Naphthylalkyl und R₅₁ und R₅₂, die an den Stickstoff der α-Aminogruppe an der N-terminalen Aminosäure gebunden sind, unabhängig voneinander für H, C₁₋₁₂Alkyl, C₇₋₁₀Phenylalkyl oder COE₂₄ stehen, wobei E₂₄ C₁₋₂₀Alkyl, C₃₋₂₀Alkenyl, C₃₋₂₀Alkinyl, Phenyl, Naphthyl oder C₇₋₁₀Phenylalkyl bedeuten, vorausgesetzt daß der jeweils andere Rest Wasserstoff ist, wenn einer der Reste R₅₁ oder R₅₂ COE₂₄ ist; und des weiteren vorausgesetzt, daß es sich bei mindestens einem der Reste R₅₆, R₅₈ und R₆₂ oder R₆₄ um CH₂-NH handelt; oder ein pharmazeutisch nutzbares Salz davon.

3. Analoges der Substanz P nach Anspruch 1 mit der Formel H-Arg-Pro-Lys-Pro-Gln-Gln-Phe-Phe-Gly-Leu^{L}[CH₂-NH]Leu-NH₂; oder ein pharmazeutisch nutzbares Salz davon.

4. Analoges der Substanz P nach Anspruch 2 mit der Formel H-D-Arg-Pro-Lys-Pro-Gln-Gln-D-Trp-Phe-D-Trp^{L}[CH₂-NH]Leu-Nle-NH₂; oder ein pharmazeutisch nutzbares Salz davon.

5. Analoges der Substanz P nach Anspruch 2 mit der Formel H-D-Arg-Pro-Lys-Pro-Gln-Gln-D-Trp-Phe-D-Trp-Leu^{L}[CH₂-NH]Nle-NH₂; oder ein pharmazeutisch nutzbares Salz davon.

6. Analoges der Substanz P nach Anspruch 2 mit der Formel H-D-Arg-Pro-Lys-Pro-Gln-Gln-D-Trp^{L}[CH₂-NH]Phe-D-Trp-Leu-Nle-NH₂; oder ein pharmazeutisch nutzbares Salz davon.

7. Analoges der Substanz P nach Anspruch 2 mit der Formel H-D-Arg-Pro-Lys-Pro-Gln-Gln-L[CH₂-NH]D-Trp-Phe-D-Trp-Leu-Nle-NH₂; oder ein pharmazeutisch nutzbares Salz davon.

8. Analoges der Substanz P nach Anspruch 1, worin
A¹ = das D- oder L-lsomere von Arg;
A² = Pro;
A³ = Lys;
A⁴ = Pro;
A⁵ = Gln;
A⁶ = Gln;
A⁷ = Phe oder D-Trp;
A⁸ = die charakterisierende Gruppe von Phe;
A⁹ = Gly oder D-Trp;
A¹⁰ = die charakterisierende Gruppe von Leu; und
A¹¹ = die charakterisierende Gruppe von Leu oder Nle.

9. Analoges der Substanz P nach Anspruch 2, worin
A²⁹ = die charakterisierende Gruppe von D-Trp.

10. Analoges der Substanz P nach Anspruch 2, worin
A²¹ = das D- oder L-lsomere von Arg;
A²² = Pro;
A²³ = Lys;
A²⁴ = Pro;
A²⁵ = die charakterisierende Gruppe von Gln;
A²⁶ = die charakterisierende Gruppe von Gln;
A²⁷ = die charakterisierende Gruppe von Phe oder D-Trp;
A²⁸ = die charakterisierende Gruppe von Phe;
A²⁹ = die charakterisierende Gruppe von D-Trp;
A³⁰ = die charakterisierende Gruppe von Leu; und
A³¹ = die charakterisierende Gruppe von Leu oder Nle.

## Revendications

1. Analogue de la substance P, ayant la formule 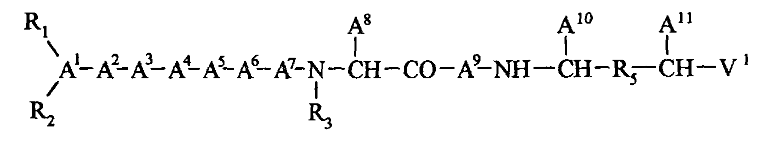 dans laquelle
A¹ = l'isomère D ou L d'Arg ou Lys;
A² = l'isomère D ou L de Pro;
A³ = l'isomère D ou L de Lys;
A⁴ = l'isomère D ou L de Pro;
A⁵ = l'isomère D ou L de Gln;
A⁶ = l'isomère D ou L de Gln;
A⁷ = l'isomère D ou L de Phe, Trp, β-Nal, o-X-Phe où X est F, Cl, Br, NO₂, OH ou CH₃, ou de p-X-Phe où X est F, Cl, Br, NO₂, OH ou CH₃;
A⁸ = le groupe identifiant de l'isomère D ou L de Trp, β-Nal, Phe, o-X-Phe où X est F, Cl, Br, NO₂, OH ou CH₃, ou de p-X-Phe où X est F, Cl, Br, NO₂, OH ou CH₃;
A⁹ = Gly ou l'isomère D ou L de Trp, β-Nal, Phe, o-X-Phe où X est F, Cl, Br, NO₂, OH ou CH₃, ou de p-X-Phe où X est F, Cl, Br, NO₂, OH ou CH₃;
A¹⁰ = le groupe identifiant de l'isomère D ou L de Leu, Nle, Ala, Val ou Ile;
A¹¹ = le groupe identifiant de l'isomère D ou L de Leu, Nle, Ala, Val ou Ile;
R₃ est H ou un alkyle en C₁-C₁₂;
R₅ est CH₂-NH-; et 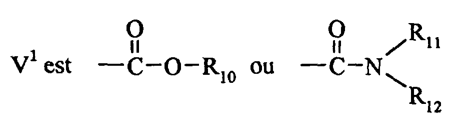
où R₁₀, R₁₁et R₁₂ sont chacun indépendamment H ou un groupe alkyle en C₁-C₁₂, phénylalkyle en C₇-C₁₀ ou naphtylalkyle en C₁₂-C₂₀; et
R₁ et R₂, qui sont liés à l'atome d'azote du groupe α-amino de l'aminoacide N-terminal sont indépendamment H ou un groupe alkyle en C₁-C₁₂, phénylalkyle en C₇-C₁₂ ou COE₁₄, E₁₄ étant un groupe alkyle en C₁-C₂₀, alcényle en C₃-C₂₀ alcynyle en C₃-C₂₀, phényle, naphtyle ou phénylalkyle en C₇-C₁₀, à condition que, lorsque l'un des R₁ et R₂ est un groupe COE₁₄, l'autre soit un atome d'hydrogène;
ou un de ses sels pharmaceutiquement acceptables.

2. Analogue de la substance P, ayant la formule 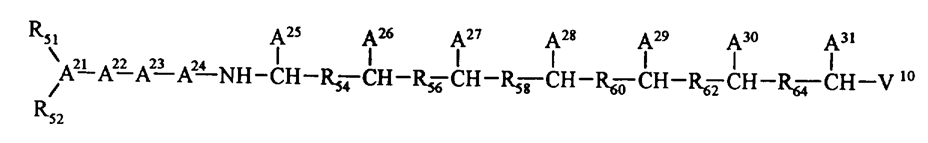 dans laquelle
A²¹ = l'isomère D ou L d'Arg ou Lys;
A²² = l'isomère D ou L de Pro;
A²³ = l'isomère D ou L de Lys;
A²⁴ = l'isomère D ou L de Pro;
A²⁵ = le groupe identifiant de l'isomère D ou L de Gln;
A²⁶ = le groupe identifiant de l'isomère D ou L de Gln;
A²⁷ = le groupe identifiant de D-Trp; ou le groupe identifiant de l'isomère D ou L de Phe;
A²⁸ = le groupe identifiant de l'isomère D ou L de l'un quelconque des aminoacides β-Nal, Phe, o-X-Phe où X est F, Cl, Br, NO₂, OH ou CH₃, ou p-X-Phe où X est F, Cl, Br, NO₂, OH ou CH₃;
A²⁹ = le groupe identifiant de l'aminoacide D-Trp; ou le groupe identifiant de l'isomère D ou L de Phe;
A³⁰ = le groupe identifiant de l'isomère D ou L de l'un quelconque des aminoacides Leu, Nle, Ala, Val ou Ile;
A³¹ = le groupe identifiant de l'isomère D ou L de l'un quelconque des aminoacides Leu, Nle, Ala, Val ou Ile;
R₅₄ est CO-NH ou CO-NCH₃;
R₅₆, R₆₂ et R₆₄ sont chacun indépendamment un groupe CO-NH ou CH₂-NH;
R₅₈ est CO-NR₆₉ où R₆₉ est H ou un alkyle en C₁-C₁₂ ou CH₂-NH;
R₆₀ est CO-NH; et
V¹⁰ est 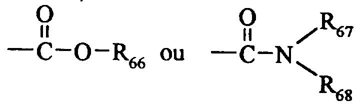
où R₆₆, R₆₇ et R₆₈ sont chacun indépendamment H ou un groupe alkyle en C₁-C₁₂, phénylalkyle en C₇-C₁₀ ou naphtylalkyle en C₁₂-C₂₀; et
R₅₁ et R₅₂, qui sont liés à l'atome d'azote du groupe α-amino de l'aminoacide N-terminal sont indépendamment H ou un groupe alkyle en C₁-C₁₂, phénylalkyle en C₇-C₁₀ ou COE₂₄, E₂₄ étant un groupe alkyle en C₁-C₂₀, alcényle en C₃-C₂₀, alcynyle en C₃-C₂₀, phényle, naphtyle ou phénylalkyle en C₇-C₁₀,
à condition que, lorsque l'un des R₅₁ et R₅₂ est un groupe COE₂₄, l'autre soit un atome d'hydrogène; et à condition qu'au moins l'un des R₅₆, R₅₈, R₆₂ et R₆₄ soit CH₂NH; ou un de ses sels pharmaceutiquement acceptables.

3. Analogue de la substance P selon la revendication 1, ayant la formule: H-Arg-Pro-Lys-Pro-Gln-Gln-Phe-Phe-Gly-Leu^{L}[CH₂-NH]Leu-NH₂; ou un de ses sels pharmaceutiquement acceptables.

4. Analogue de la substance P selon la revendication 2, ayant la formule: H-D-Arg-Pro-Lys-Pro-Gln-Gln-D-Php-Phe-D-Trp^{L}[CH₂-NH]Leu-Nle-NH₂; ou un de ses sels pharmaceutiquement acceptables.

5. Analogue de la substance P selon la revendication 2, ayant la formule: H-D-Arg-Pro-Lys-Pro-Gln-Gln-D-Php-Phe-D-Trp-Leu^{L}[CH₂-NH]Nle-NH₂; ou un de ses sels pharmaceutiquement acceptables.

6. Analogue de la substance P selon la revendication 2, ayant la formule: H-D-Arg-Pro-Lys-Pro-Gln-Gln-D-Trp^{L}[CH₂-NH]Phe-D-Trp-Leu-Nle-NH₂; ou un de ses sels pharmaceutiquement acceptables.

7. Analogue de la substance P selon la revendication 2, ayant la formule: H-D-Arg-Pro-Lys-Pro-Gln-Gln-^{L}[CH₂-NH]D-Trp-Phe-D-Trp-Leu-Nle-NH₂; ou un de ses sels pharmaceutiquement acceptables.

8. Analogue de la substance P selon la revendication 1 dans lequel
A¹ = l'isomère D ou L d'Arg;
A² = Pro;
A³ = Lys;
A⁴ = Pro;
A⁵ = Gln;
A⁶ = Gln;
A⁷ = Phe ou D-Trp;
A⁸ = le groupe identifiant de Phe;
A⁹ = Gly ou D-Trp;
A¹⁰ = le groupe identifiant de Leu; et
A¹¹ = le groupe identifiant de Leu ou Nle.

9. Analogue de la substance P selon la revendication 2, dans lequel
A²⁹ = le groupe identifiant de D-Trp.

10. Analogue de la substance P selon la revendication 2, dans lequel
A²¹ = l'isomère D ou L d'Arg;
A²² = Pro;
A²³ = Lys;
A²⁴ = Pro;
A²⁵ = le groupe identifiant de Gln;
A²⁶ = le groupe identifiant de Gln;
A²⁷ = le groupe identifiant de Phe ou de D-Trp;
A²⁸ = le groupe identifiant de Phe;
A²⁹ = le groupe identifiant de D-Trp;
A³⁰ = le groupe identifiant de Leu; et
A³¹ = le groupe identifiant de Leu ou de Nle.
